# EUROPÄISCHE PATENTANMELDUNG

(11) **EP 4 155 287 A1**
(43) Veröffentlichungstag der Anmeldung: **29.03.2023**
(21) Anmeldenummer: 21198533.8
(22) Anmeldetag: 23.09.2021
(51) Int. Cl.: C07C 31/04, B01D 53/02, B01J 8/04, C07C 29/152

(54) **REAKTOR UND VERFAHREN ZUR METHANOL-SYNTHESE**

(71) Anmelder: Clariant International Ltd, 4132 Muttenz (CH); L'Air Liquide, Société Anonyme pour l'Etude et l'Exploitation des Procédés Georges Claude, 75007 Paris (FR)
(72) Erfinder: HAAG, Stéphane, 60388 Frankfurt am Main (DE); DO, Nga Thi Quynh, 60388 Frankfurt am Main (DE); GRONEMANN, Veronika, 60439 Frankfurt am Main (DE); OELMANN, Tobias, 60439 Frankfurt am Main (DE); REICHINGER, Markus, 83052 Bruckmühl (DE); REITMEIER, Stephan J., 85614 Kirchseeon (DE); SCHWARZ, Heiner, 80799 München (DE)
(74) Vertreter: Dannenberger, Oliver Andre

(57) **Zusammenfassung**

Die Erfindung betrifft einen Reaktor zur katalytischen Herstellung von Methanol, in dem zwei oder mehr als zwei Reaktionszonen, die von einem Wärmetauschfluid umgeben sind, angeordnet sind, die jeweils mindestens eine Reinigungsschicht und mindestens eine Katalysatorschicht umfassen. Die Reinigungsschicht(en) und die Katalysatorschicht(en) sind in Reaktionszonen, bei denen es sich vorzugsweise um Reaktorrohre handelt, angeordnet, wobei die Reinigungsschicht(en) stromaufwärts und die Katalysatorschicht(en) stromabwärts angeordnet sind. Die Erfindung betrifft ferner ein Verfahren zur katalytischen Herstellung von Methanol aus Synthesegas unter Verwendung des Reaktors.

## Beschreibung

Die Erfindung betrifft einen Reaktor zur katalytischen Herstellung von Methanol sowie ein Verfahren zur katalytischen Herstellung von Methanol aus Synthesegas.

### Hintergrund der Erfindung

Verfahren zur Herstellung von Methanol durch katalytische Umsetzung von Wasserstoff und Kohlenstoffoxide enthaltendem Synthesegas sind der Fachwelt seit langer Zeit bekannt. So wird in Ullmann's Encyclopedia of Industrial Chemistry, Sixth Edition, 1998 Electronic Release, Kapitel "Methanol", Unterkapitel 5.2 "Synthesis" ein Verfahren zur Herstellung von Methanol beschrieben. Die bekannte Synthese von Methanol aus Synthesegas, das üblicherweise Kohlenstoffmonoxid, Kohlenstoffdioxid und Wasserstoff umfasst, kann durch die folgenden Gleichungen beschrieben werden:
1)

   *CO*+2*H*₂ ⇄ *CH*₃*OH;*
2)

   *CO*₂ + 3*H*₂ ⇄ *CH*₃*OH* + *H*₂*O*;
3)

   *CO*+*H*₂O ⇄ *H*₂+*CO*₂

Diese drei Reaktionen sind exotherm. In den Reaktionen 1 und 2 wird Methanol gebildet, niedrige Temperaturen und Druckerhöhung führen zu einer Verschiebung des Gleichgewichtes zum gewünschten Produkt, Methanol. Bei der simultan stattfindenden, ebenfalls exothermen Reaktion 3 handelt es sich um die sogenannte "Wassergas Shift" Reaktion, bei welcher Kohlenstoffmonoxid in Kohlenstoffdioxid umgewandelt wird. An Stelle einer Mischung von Kohlenstoffmonoxid, Kohlenstoffdioxid und Wasserstoff, das üblicherweise als "Synthesegas" bezeichnet wird, kann als Edukt auch ein Gasgemisch aus Kohlenstoffdioxid und Wasserstoff eingesetzt werden. Aufgrund der Reversibilität der "Wassergas Shift"-Reaktion kann Kohlenstoffmonoxid gebildet werden. Insbesondere bei einer Rückführung nicht umgesetzter Gasbestandteile (sogenannte Syntheseschleife) kann es dadurch zur Anreicherung von Kohlenstoffmonoxid kommen, obwohl im ursprünglichen Edukt gar kein Kohlenmonoxid enthalten war.

Katalytische Reaktionen im industriellen Maßstab verlaufen im Allgemeinen unter Bedingungen, bei denen der eingesetzte Katalysator über die Dauer der Verwendung zunehmend deaktiviert wird. Eine Deaktivierung kann zum Beispiel aufgrund einer hydrothermalen Alterung und/oder durch eine Vergiftung der Katalysatoren durch Katalysatorgifte im Zuführungsgasstrom erfolgen. Selbst wenn der Zuführungsgasstrom vor der Einleitung in den Reaktor einem Reinigungsprozess unterzogen wird, bleiben typischerweise geringe Mengen an Verunreinigungen enthalten. Von besonderer Bedeutung sind Spuren von Schwefel-, Chlor-, Halogen-, Eisen- und Nickel-Verbindungen und ungesättigten Kohlenwasserstoffen, die im Zuführungsgasstrom vorhanden sein können, als Katalysatorgifte wirken und die Lebensdauer und somit die über die Lebensdauer der Katalysatoren erzielbare Methanolproduktion, d.h. die "Lifetime-Yield", negativ beeinflussen können.

Beispielsweise ist bekannt, dass Metalle Sulfide bilden können, selbst wenn Schwefel als Verunreinigung im Zuführungsstrom nur in geringen Mengen vorhanden ist. Dies betrifft im Prinzip alle katalytischen Verfahren, bei denen Metall- oder Edelmetallkatalysatoren zum Einsatz kommen, wie zum Beispiel
Ammonoxidationsreaktionen, Dehydrierungsreaktionen, katalytische Reformierungsreaktionen und Oxidationsreaktionen, insbesondere die Methanolsynthese einschließt.

Insbesondere bei der Methanolsynthese ausgehend von einem Synthesegasstrom können Carbonyle wie Fe(CO)₅ oder Ni(CO)₄ vorhanden sein. Diese können bereits als Nebenprodukte bei der Gewinnung des Synthesegases aus Kohle entstehen oder durch die Reaktion von Kohlenstoffmonoxid, das über den Synthesegasstrom zugeführt wird oder sich innerhalb einer Syntheseschleife bildet, mit den Edelstahlverkleidungen der Leitungswände entstehen. Bei Kontakt mit dem Katalysator für die Methanolsynthese - typischerweise ein Katalysator auf Cu/Zn/AI-Basis - werden diese Carbonyle von diesem adsorbiert und zerfallen im Weiteren in CO und das entsprechende Metall, wodurch die katalytische Aktivität des Katalysators drastisch reduziert wird und/oder es zu einer erhöhten Bildung von unerwünschten Nebenprodukten kommt. Dies kann dazu führen, dass der Katalysator früher ersetzt werden muss. Die Deaktivierung von Methanolkatalysatoren durch Eisen ist seit langem bekannt und ist beispielsweise in Harald H. Kung, Catal. Today, 1992, 11, S. 443-453 beschrieben. Aufgrund des hohen Gasdurchsatzes bei der katalytischen Methanolsynthese aus Synthesegas können bereits geringe Konzentrationen, beispielsweise im ppb-Bereich, an Carbonylen wie Fe(CO)₅ ausreichen, um die Standzeit des Katalysators signifikant zu reduzieren.

Zur Vermeidung bzw. Verringerung einer solchen Deaktivierung der eingesetzten Katalysatoren durch Katalysatorgifte kann eine Reinigung des Synthesegases der eigentlichen Synthesereaktion vorgeschaltet werden. Dabei kommen Reinigungsmaterialien (auch bekannt als Guardbett-Materialen) zum Einsatz, die die Fähigkeit aufweisen, mögliche Katalysatorgiften durch Adsorption, Absorption und/oder Zersetzung zu entfernen.

Zu typischen Katalysatorgiften bei der Methanolsynthese gehören unter anderem Schwefel- und Chlorverbindungen. In der GB 1 357 335 wird vorgeschlagen, stromaufwärts eines "Wassergas-Shift"-Katalysators auf Kupferbasis ein Guardbett vorzusehen, das auf Aluminiumoxid geträgerte Oxide von Alkalimetallen, Erdalkalimetallen, Mangan, Yttrium oder Lanthan enthält. Die WO 01/17674 beschreibt ein Guardbett, das ein Chlorid-Absorptionsmittel auf Bleibasis sowie einen Träger dafür enthält.

Die US 5,451,384 A beschreibt die Abtrennung von Metallcarbonylen aus einem Gasstrom, zum Beispiel einem Synthesegasstrom, durch Adsorption an Bleioxid auf einem Trägermaterial. Die EP 2 069 231 A1 offenbart ein Verfahren zur Abtrennung von Metallcarbonylen aus einem Synthesegasstrom unter Verwendung eines Adsorbens, das Aktivkohle oder einen hydrophoben Zeolithen aufweist.

Die WO 2008/144402 beschreibt ein Verfahren zur Epoxidierung von Olefinen, insbesondere von Ethylen. Darin wird ein Reaktorsystem beschrieben, das eine oder mehrere Reinigungszonen und eine Reaktionszone umfasst, wobei die Reaktionszone innerhalb eines oder mehrerer offener Reaktorrohre eines Rohrbündel-Wärmetauscher-Reaktorgefäßes angeordnet ist. Die Reinigungszonen können entweder in einem separaten Reaktorgefäß stromaufwärts des Rohrbündel-Wärmetauscher-Reaktorgefäßes oder innerhalb des Reaktorgefäßes angeordnet sein, wobei die Reinigungszone im letztgenannten Fall stromaufwärts der Reaktorrohre positioniert ist, beispielsweise im Kopfraum des Reaktorgefäßes. In beiden Fällen ist eine zusätzliche, von der Temperierung der Reaktorrohre unabhängige Temperierung der Reinigungszonen erforderlich, um eine für die Reinigungswirkung des Absorptionsmittels optimale Temperatur gewährleisten zu können. Zudem weisen nicht alle gängigen Reaktoren genügend Platz im Kopfraum des Reaktorgefäßes auf, um darin Reinigungsmaterial in hinreichender Menge zu platzieren. Außerdem kann das durch die Eintrittsöffnung des Reaktorgefäßes einströmende Gas zu einer Verwirbelung des Absorptionsmittels führen.

Neben einer möglichen Deaktivierung des Katalysators durch Katalysatorgifte in den Zuführungsgasen besteht ein weiteres Problem darin, dass bei der Verwendung von hochaktivem Katalysatormaterial und einer entsprechend hohen Umsetzung des Synthesegases viel Wärme frei wird. Daher erfolgt die Methanolsynthese im Allgemeinen in einem Rohrbündelreaktor mit Wärmetauscher, der eine Vielzahl von Reaktionsrohren umfasst, die jeweils ein gepacktes Bett aus Katalysator enthalten und von einem Wärmetauschfluid, wie einem Heiz- oder Kühlfluid, umgeben sind. Dennoch hat sich bei der Verwendung von Katalysatoren mit hoher Aktivität gezeigt, dass sich beim Starten des Reaktors im Anfangsbereich des Reaktorbettes eine ausgeprägte nicht-isotherme Reaktionszone mit ausgeprägtem Temperaturgradienten und einem lokalen Temperaturmaximum (Hot-Spot) bildet. Dieser Temperaturanstieg und damit die Hot-Spot-Temperatur kann bis zu 70 K oberhalb der Gas-Eintrittstemperatur oder bis zu 40 K über der Kühlmitteltemperatur liegen. Darüber hinaus führt die erhöhte Temperatur zu einer schnelleren Deaktivierung des Katalysatormaterials durch Sintern. Die Deaktivierung des Katalysators wiederum sorgt für ein Wandern des Hot-Spots durch den Reaktor. Eine hohe Temperatur führt daneben auch dazu, dass das thermodynamische Gleichgewicht der Reaktion erreicht wird. Beim Erreichen des thermodynamischen Gleichgewichts findet kein weiterer Umsatz des Synthesegases mehr statt. Im stromabwärtigen Teil des Katalysatorbetts liegen dann niedrigere Temperaturen sowie ein sinkender Anteil an Kohlenstoffoxiden im Synthesegas vor, da ein Teil der Oxide bereits umgesetzt wurde.

Es ist daher eine Aufgabe der vorliegenden Erfindung ein Verfahren zur Herstellung von Methanol bereitzustellen sowie hierin verwendete Reaktoren derart weiterzubilden, dass die oben beschriebenen Nachteile vermieden oder zumindest verringert werden und die Raum-Zeit-Ausbeute gesteigert werden kann oder zumindest beibehalten wird. Insbesondere ist die Aufgabe der vorliegenden Erfindung ein Verfahren zur Herstellung von Methanol sowie dazu verwendete Reaktoren bereitzustellen, wodurch eine Verlängerung der Lebensdauer eines Methanol-Katalysators unter Anwesenheit von Katalysatorgiften und hydrothermaler Alterung bei gleichzeitiger Beibehaltung und idealerweise sogar Erhöhung der Methanolausbeute ermöglicht wird.

Die Erfinder der vorliegenden Erfindung haben überraschenderweise festgestellt, dass diese Aufgabe gelöst wird, indem man in einem Rohrreaktor mit Wärmetauscher eine stromaufwärtige Reinigungsschicht und wenigstens eine stromabwärtige Katalysatorschicht innerhalb der Reaktorrohre bereitstellt. Durch diese Anordnung kann die Temperatur des zugeführten Synthesegases über die Wärmeaustauschflüssigkeit vorgewärmt werden. Die Vorwärmung des zugeführten Synthesegases wirkt sich sowohl auf die Reinigungswirkung der Reinigungsschicht als auch auf die Umsetzung des Synthesegases in einer stromabwärtigen Katalysatorschicht positiv aus. Die Vorteile der vorliegenden Erfindung stellen sich auch ein, wenn anstelle eines Rohrreaktors mit Wärmetauscher ein Reaktor mit einem oder mehreren Thermoplatten verwendet wird. Derartige Reaktoren werden beispielweise in der EP 1 147 807 A, der EP 3 401 299 A1 und der DE 10 2004 036 695 A1 beschrieben. Die Reaktion wird dabei in den Zwischenräumen zwischen den Thermoplatten eines derartigen Reaktors durchgeführt, die im Stand der Technik mit festem Katalysator gefüllt sind. Durch das erfindungsgemäße Bereitstellen einer stromaufwärtigen Reinigungsschicht und wenigstens einer stromabwärtigen Katalysatorschicht innerhalb der Zwischenräume zwischen Thermoplatten können die vorstehend beschriebenen Vorteile der vorliegenden Erfindung ebenfalls realisiert werden.

### Zusammenfassung der Erfindung

Die Erfindung betrifft einen Reaktor zur katalytischen Herstellung von Methanol, umfassend zwei oder mehr als zwei Reaktionszonen, die von einem Wärmetauschfluid umgeben sind, wobei in den zwei oder den mehr als zwei Reaktionszonen jeweils wenigstens eine Katalysatorschicht angeordnet ist und in den zwei oder den mehr als zwei Reaktionszonen jeweils wenigstens eine Reinigungsschicht angeordnet ist, die ein Reinigungsmaterial umfasst, und wobei die wenigstens eine Reinigungsschicht stromaufwärts der wenigstens einen Katalysatorschicht angeordnet ist. Bei den Reaktionszonen handelt es sich vorzugsweise um Reaktorrohre.

Außerdem betrifft die Erfindung ein Verfahren zur katalytischen Herstellung von Methanol aus Synthesegas, wobei das Verfahren folgende Schritte umfasst:
(a) Bereitstellen eines Reaktors, vorzugsweise eines Wärmetauscher-Reaktors,
(b) Anordnen von zwei oder mehr als zwei Reaktionszonen im Reaktor,
(c) Beaufschlagen des Reaktors mit Synthesegas, umfassend Wasserstoff und Kohlenstoffoxide,
(d) Umsetzen des Synthesegases in dem Reaktor unter Methanolsynthesebedingungen zu Methanol, und
(e) Ausleiten des erzeugten Methanols und des nicht umgesetzten Synthesegases aus dem Reaktor,

wobei in den zwei oder den mehr als zwei Reaktionszonen jeweils wenigstens eine Katalysatorschicht angeordnet ist, und
wobei in den zwei oder den mehr als zwei Reaktionszonen jeweils wenigstens eine Reinigungsschicht angeordnet ist, die ein Reinigungsmaterial umfasst, und wobei die wenigstens eine Reinigungsschicht stromaufwärts der wenigstens einen Katalysatorschicht angeordnet ist.

### Detaillierte Beschreibung der Erfindung

In einer ersten Ausführungsform betrifft die Erfindung einen Reaktor zur katalytischen Herstellung von Methanol. Der Reaktor umfasst zwei oder mehr als zwei Reaktionszonen, die von einem Wärmetauschfluid umgeben sind. Die Reaktionszonen sind vorzugsweise durch Reaktorrohre ausgebildet

Alternativ können die Reaktionszonen auch unter Verwendung von Thermoplatten ausgebildet werden, die von Wärmetauschfluid durchströmt werden. Eine Thermoplatte im Sinne der vorliegenden Erfindung besteht aus zwei Blechen, welche an den Rändern verbunden, vorzugsweise zusammengeschweißt sind, und über deren Oberfläche eine Vielzahl von zusätzlichen Verbindungen, vorzugsweise Punktschweißungen, die die Bleche ebenfalls miteinander verbinden, verteilt ist. Solche Bleche können automatisiert von Robotern oder Maschinen und somit zu sehr günstigen Preisen hergestellt werden. Nach der Verschweißung werden die erhaltenen Thermoplatten durch eine hydraulische Umformung, in der Regel durch Einpressen einer Flüssigkeit unter hohem Druck, expandiert, wodurch kissenartige Kanäle zwischen den die Thermoplatten bildenden Blechen entstehen, durch die ein Wärmetauschfluid, wie ein Heiz- oder Kühlfluid geleitet werden kann. Über die Wärmetransporträume kann daher bestimmten Bereichen des Reaktors durch das Durchleiten von Wärmetauschfluid sowohl Wärmeenergie zu- als auch abgeführt werden. Dazu werden in einem Reaktor zunächst drei oder mehr als drei Thermoplatten im Wesentlichen parallel zueinander und zur Längsrichtung angeordnet. Im Wesentlichen parallel im Sinne der Erfindung bedeutet, dass die Ausrichtung der Thermoplatten zueinander und zur Längsrichtung von der Parallelen um +/- 20° oder weniger, bevorzugt um +/- 10° oder weniger, besonders bevorzugt um +/- 5° oder weniger, ganz besonders bevorzugt um +/- 2° oder weniger voneinander abweicht. Die Zwischenräume oder Freiräume zwischen jeweils zwei benachbarten Thermoplatten bilden in dieser Ausführungsform die Reaktionszonen.

Eine "Reaktionszone" im Sinne der vorliegenden Erfindung kann daher als Reaktorrohr oder als Zwischenraum zwischen zwei benachbarten Thermoplatten ausgestaltet sein. Vorzugsweise sind die Reaktionszonen als Reaktorrohre ausgebildet.

In den zwei oder mehr als zwei Reaktionszonen sind jeweils wenigstens eine Katalysatorschicht und jeweils wenigstens eine Reinigungsschicht angeordnet. Die Reinigungsschicht umfasst ein Reinigungsmaterial und ist in den Reaktionszonen stromaufwärts angeordnet. Die Katalysatorschicht(en) ist/sind in den Reaktionszonen stromabwärts angeordnet.

Die Begriffe "stromaufwärts" oder "stromabwärts" bezeichnet in der vorliegenden Erfindung die Anordnung der Reinigungs- und Katalysatorschichten im Reaktor. Im Reaktor sind eine Gaseintrittsseite sowie eine Gasaustrittsseite für Synthesegas vorgesehen. Das Synthesegas tritt durch die Gaseintrittsseite in den Innenraum des Reaktors ein, in dem die Katalysatorschichten vorgesehen sind. Nach dem Durchströmen der Katalysatorschichten tritt das Synthesegas auf der Gasaustrittsseite aus dem Reaktor aus. Der Strom an Synthesegas definiert somit eine Richtung, wobei das Synthesegas durch die "stromaufwärts" gelegene Gaseintrittsseite in den Innenraum des Reaktors eintritt und durch die "stromabwärts" gelegene Gasaustrittsseite aus dem Innenraum des Reaktors austritt. Die Reinigungsschicht befindet sich somit im Reaktor näher an der Gaseintrittsseite als die erste Katalysatorschicht, die sich näher an der Gasaustrittsseite des Reaktors befindet.

In einer bevorzugten Ausführungsform umfasst der Reaktor 2 bis 25000 Reaktionszonen, stärker bevorzugt 1000 bis 20000 Reaktionszonen, insbesondere 2500 bis 15000 Reaktionszonen.

Vorzugsweise ist der Reaktor als ein Rohrbündelreaktor ausgebildet, der 2 bis 25000 Reaktorrohre, stärker bevorzugt 1000 bis 20000 Reaktorrohre, insbesondere 2500 bis 15000 Reaktorrohre enthält.

In einer weiteren Ausführungsform ist der Reaktor unter Verwendung von Thermoplatten ausgebildet, wobei der Reaktor 3 bis 10000 Thermoplatten, bevorzugt 100 bis 8000 Thermoplatten, insbesondere 500 bis 5000 Thermoplatten enthält.

Die Reaktionszonen sind vorzugsweise im Wesentlichen parallel zur zentralen Längsachse des Reaktors angeordnet.

In einer bevorzugten Ausführungsform wird als Wärmetauschfluid Wasser bzw. Wasserdampf, ein Wärmeträgeröl, wie beispielsweise Dowtherm A, oder ein geeignetes Gas eingesetzt. Vorzugsweise wird als Wärmetauschfluid bzw. Kühlmittelfluid Wasser bzw. Wasserdampf verwendet. Das heißt, dass der Reaktor vorzugsweise als wassergekühlter, ölgekühlter oder als gasgekühlter Reaktor, insbesondere als wassergekühlter Reaktor ausgebildet ist.

Die Reaktorrohre können einen Innendurchmesser im Bereich von 10 bis 80 mm, vorzugsweise im Bereich von 20 bis 75 mm und besonders bevorzugt im Bereich von 25 bis 70 mm aufweisen. Die Reaktorrohre können eine Länge im Bereich von 0,5 bis 25 m, vorzugsweise im Bereich von 2 bis 15 m und besonders bevorzugt im Bereich von 5 bis 10 m haben.

In der Ausführungsform, bei der die Reaktionszonen unter Verwendung von Thermoplatten ausgebildet werden, sind die Abstände zwischen jeweils zwei Thermoplatten typischerweise im Bereich von 10 mm bis 100 mm, vorzugsweise im Bereich von 20 bis 50 mm. Der Abstand bezieht sich dabei auf den Abstand von Mittellinie zur Mittellinie, wobei die Mittellinie die Symmetrieachse der aus zwei Blechen gebildeten Thermoplatten bezeichnet. Der Abstand kann an die Abmessungen der Katalysatorpartikel angepasst werden, um eine optimale Wärmeabfuhr sowie ein gutes Schüttgutverhalten beim Befüllen und Entleeren des Katalysators sicherzustellen.

In den Reaktionszonen, die in einer bevorzugten Ausführungsform als Reaktorrohre ausgebildet sind, ist jeweils wenigstens eine Katalysatorschicht angeordnet, die einen oder mehrere Katalysatoren umfasst. Es können auch zwei oder mehr Katalysatorschichten in den Reaktionszonen angeordnet sein. Die Katalysatorschichten sind vorzugsweise, in Strömungsrichtung des Synthesegases, direkt aneinander angrenzend angeordnet.

In einer bevorzugten Ausführungsform sind in den zwei oder mehr als zwei Reaktionszonen, bei denen es sich vorzugsweise um Reaktorrohre handelt, jeweils wenigstens zwei voneinander verschiedene Katalysatorschichten angeordnet. Die erste Katalysatorschicht ist stromaufwärts angeordnet und die zweite Katalysatorschicht ist stromabwärts angeordnet. Vorzugsweise ist die Aktivität der ersten Katalysatorschicht verschieden von der Aktivität der zweiten Katalysatorschicht.

Die Aktivität einer Katalysatorschicht bezeichnet hierbei den Umsatzgrad von Edukten zu Produkten in der Katalysatorschicht, normiert auf die Höhe der Katalysatorschicht. Die Aktivität einer ersten Katalysatorschicht ist daher niedriger als die Aktivität einer davon verschiedenen zweiten Katalysatorschicht, wenn bei gleicher Höhe (d.h. bei gleichem Schüttvolumen) der beiden Katalysatorschichten und unter gleichen Reaktionsbedingungen der Umsatzgrad von Edukten zu Produkten der ersten Katalysatorschicht niedriger ist als der der zweiten Katalysatorschicht.

Die Aktivität eines Katalysators kann zum Beispiel durch die chemische Zusammensetzung des Katalysatormaterials, wie durch Zugabe eines aktivitätsmoderierenden Promotors, durch Variation der verfügbaren Oberfläche etc. beeinflusst werden. Vorzugsweise sind die Katalysatoren fest. Die Katalysatorschichten können aus Katalysatoren in Pulverform und/oder aus Katalysatoren in Form von Formkörpern, insbesondere gepressten Formkörpern, wie zum Beispiel gepressten Tabletten oder gepressten Ringen, Pellets oder aus Extrudaten gebildet werden.

Die Formkörper können verschiedene Geometrien, wie Kugeln, Zylinder oder Hohlkörper wie Ringe aufweisen. Kugelförmige Pellets können einen Durchmesser d von 1 mm bis 12 mm, bevorzugt von 2 mm bis 10 mm und besonders bevorzugt von 3 mm bis 8 mm aufweisen. Extrudate, zylinderförmige Pellets bzw. gepresste Tabletten oder Ringe können einen Durchmesser d von 1 mm bis 12 mm, bevorzugt von 2 mm bis 10 mm und besonders bevorzugt von 3 mm bis 8 mm und eine Höhe h von 1 mm bis 10 mm, bevorzugt von 2 mm bis 8 mm und besonders bevorzugt von 3 mm bis 6 mm aufweisen.

Die Geometrie und Größe von Pellet-Katalysatoren kann in verschiedenen Katalysatorschichten gleich oder verschieden sein.

Beispielsweise kann in einer Katalysatorschicht, die aus Pellets in Form von kleinen Kugeln gebildet wird, eine größere Menge an Edukt zum Produkt umgesetzt werden als in einer entsprechenden Katalysatorschicht des gleichen Materials mit der gleichen Höhe, die aus Pellets in Form von größeren Kugeln gebildet wird.

Um unterschiedliche Aktivitäten zu realisieren, können in einer Katalysatorschicht kleinere zylinderförmige Pellets, beispielsweise mit 3 mm Durchmesser und 3 mm Höhe, verwendet werden, während in einer anderen Katalysatorschicht größere zylinderförmige Pellets des gleichen Materials, beispielsweise mit 6 mm Durchmesser und 4 mm Höhe, eingesetzt werden. In einer Ausführungsform werden in einer Katalysatorschicht kleinere zylinderförmige Pellets mit 3 mm Durchmesser und 3 mm Höhe verwendet, während größere zylinderförmige Pellets des gleichen Materials mit 6 mm Durchmesser und 4 mm Höhe in einer anderen Katalysatorschicht eingesetzt werden.

Vorzugsweise sind die Katalysatorschichten aus Katalysatoren in Pelletform ausgebildet, wobei es besonders bevorzugt ist, dass sich insbesondere für Katalysatorschichten unterschiedlicher Aktivität die Pelletgröße der Katalysatoren in unterschiedlichen Katalysatorschichten unterscheidet.

In einer Ausführungsform weisen die Katalysatoren der einzelnen Katalysatorschichten unterschiedliche Zusammensetzungen auf. Beispielsweise können sich die Katalysatoren der einzelnen Katalysatorschichten in der Anwesenheit oder Abwesenheit von Promotoren oder deren Mengen unterscheiden.

Vorzugsweise sind die Katalysatoren in Form von Festbett-Schüttungen angeordnet. Die Katalysatorschichten werden dabei dadurch ausgebildet, dass nacheinander die gewünschten Katalysatorschichten in die Reaktorrohre geladen werden. Dadurch grenzen die Katalysatorschichten direkt aneinander an. Bei der Beladung wird sichergestellt, dass die Katalysatorschichten in der Strömungsrichtung des Synthesegases nacheinander angeordnet werden.

In einer bevorzugten Ausführungsform ist die Aktivität der ersten Katalysatorschicht niedriger als die Aktivität der weiteren Katalysatorschicht beziehungsweise als die Aktivität der weiteren Katalysatorschichten. Alternativ dazu kann auch vorgesehen sein, dass die Aktivität der ersten Katalysatorschicht höher ist als die Aktivität der zweiten Katalysatorschicht, und die Aktivität der weiteren Katalysatorschicht(en), falls vorhanden, jeweils sukzessive ansteigt.

In einer besonders bevorzugten Ausführungsform sind in den Reaktorrohren eine oder mehrere weitere Katalysatorschichten, vorzugsweise zwei oder mehr als zwei weitere Katalysatorschichten, angeordnet, wobei die weiteren Katalysatorschichten jeweils stromabwärts der zweiten Katalysatorschicht angeordnet sind und wobei die Aktivität der weiteren Katalysatorschichten zum stromabwärts liegenden Ende des Reaktors sukzessive ansteigt.

Die Schichthöhe der ersten Katalysatorschicht beträgt vorzugsweise 20 bis 80%, bezogen auf die Gesamthöhe aller Reinigungsschichten und Katalysatorschichten in der Reaktionszone. Mit zunehmender Nutzungsdauer kann es zu einer Schrumpfung von Katalysatoren und damit zu einer Verringerung der Höhe der Katalysatorschicht(en) kommen. Die angegebenen relativen Schichthöhen der Katalysatorschichten sind so zu verstehen, dass sie sich auf den Ausgangszustand der mit Reinigungsmaterial und Katalysator beladenen Reaktionszonen beziehen, d.h. auf den Zustand vor dem Anfahren des Reaktors.

Als Katalysatoren können kupferbasierte Methanolsynthese-Katalysatoren, insbesondere Cu/Zn/Al-Katalysatoren verwendet werden.

Die verwendeten kupferbasierten Methanolsynthese-Katalysatoren können unterschiedlichen Kupferdispersionen und unterschiedliche Aktivitätsgrade aufweisen. Die Dispersion ist dabei ein Maß für die Teilchengröße der im Katalysator vorliegenden Kupferteilchen und kann beispielsweise durch CO-Chemisorption und anschließender Methanisierung des metall-gebundenen CO bestimmt werden.

In den Reaktionszonen, bei denen es sich vorzugsweise um Reaktorrohre handelt, ist jeweils wenigstens eine Reinigungsschicht angeordnet, die ein oder mehrere Reinigungsmaterialien umfasst. Die wenigstens eine Reinigungsschicht ist stromaufwärts der wenigstens einen Katalysatorschicht angeordnet. Es können auch zwei oder mehr Reinigungsschichten in den Reaktionszonen, bei denen es sich vorzugsweise um Reaktorrohre handelt, angeordnet sein. Die Reinigungsschicht(en) sind vorzugsweise, in Strömungsrichtung des Synthesegases, direkt aneinander angrenzend und an die wenigstens eine Katalysatorschicht angrenzend angeordnet.

In einer bevorzugten Ausführungsform ist eine zusätzliche Reinigungsschicht stromaufwärts, d.h. außerhalb der zwei oder mehr als zwei Reaktionszonen, bei denen es sich vorzugsweise um Reaktorrohre handelt, angeordnet. Die optionale zusätzliche Reinigungsschicht ist vorzugsweise innerhalb des Reaktors angeordnet. Die optionale zusätzliche Reinigungsschicht weist ein Reinigungsmaterial auf, das gleich oder verschieden ist vom Reinigungsmaterial in den zwei oder den mehr als zwei Reaktionszonen, bei denen es sich vorzugsweise um Reaktorrohre handelt.

Die Schichthöhe der Reinigungsschicht(en) in den Reaktionszonen, bei denen es sich vorzugsweise um Reaktorrohre handelt, beträgt vorzugsweise 5 bis 30 %, bevorzugt 5 bis 20 %, stärker bevorzugt 7,5 bis 20 %, besonders bevorzugt 10 bis 15 % bezogen auf die Gesamtschichthöhe aller Reinigungsschicht(en) und Katalysatorschichten in der Reaktionszone. Die angegebenen relativen Schichthöhen der Reinigungsschicht(en) sind so zu verstehen, dass sie sich auf den Ausgangszustand der mit Reinigungsmaterial und Katalysator beladenen Reaktionszonen, insbesondere den Ausgangszustand der mit Reinigungsmaterial und Katalysator beladenen Reaktorrohre beziehen, d.h. auf den Zustand vor dem Anfahren des Reaktors.

Das Reinigungsmaterial zeichnet sich dadurch aus, dass es in der Lage ist, die Menge an Verunreinigungen zu reduzieren, die in einem Synthesegasstrom vorhanden sind und die den verwendeten Katalysator unter den vorherrschenden Bedingungen vergiften können.

Die Reinigungsschicht(en) sind zu Beginn der Verwendung im Reaktor vorzugsweise in der Lage aus dem Synthesegasstrom die Menge an Verringerungen, bezogen auf deren Menge im eingesetzten Synthesegasstrom, um 10 Gewichtsprozent oder mehr, stärker bevorzugt um 25 Gewichtsprozent oder mehr, besonders bevorzugt um 50 Gewichtsprozent oder mehr, ganz besonders bevorzugt um 90 Gewichtsprozent oder mehr zu reduzieren. Die Reinigungsschicht(en) sind zu Beginn der Verwendung im Reaktor vorzugsweise in der Lage aus dem Synthesegasstrom die Menge an Verringerungen, bezogen auf deren Menge im eingesetzten Synthesegasstrom, um 1 Gewichtsprozent oder mehr, stärker bevorzugt um 5 Gewichtsprozent oder mehr, besonders bevorzugt um 25 Gewichtsprozent oder mehr, ganz besonders bevorzugt um 40 Gewichtsprozent oder mehr zu reduzieren.

Die Verunreinigungen, deren Menge im Synthesegasstrom durch das Reinigungsmaterial verringert wird, sind insbesondere Metalle und deren Verbindungen, wie elementares Eisen, Eisen-haltige Verbindungen, insbesondere Eisen-Carbonyle, und Nickel-haltige Verbindungen, insbesondere Nickel-Carbonyle, schwefelhaltige Verbindungen, wie Sulfide oder Sulfate, ungesättigte Kohlenwasserstoffe, chlorhaltige Verbindungen, und Mischungen davon.

Die Verringerung der Menge an Verunreinigungen kann über eine Adsorption und/oder Absorption der Verunreinigungen an das Reinigungsmaterial und/oder durch eine Zersetzung der Verunreinigungen erfolgen. Unter Adsorption im Sinne der vorliegenden Erfindung ist zu verstehen, dass Stoffe, insbesondere Moleküle auf der Oberfläche des Reinigungsmittels haften bleiben und sich auf dessen zugänglicher Oberfläche anreichern, wobei die Anhaftung im Wesentlichen nicht über chemische Bindungen, sondern über Van-der-Waals-Kräfte erfolgt. Demgegenüber bezeichnet der Begriff Absorption den Vorgang, bei dem eine Anhaftung von Stoffen, insbesondere von Molekülen an die zugängliche Oberfläche des Reinigungsmittels durch Ausbildung chemischer Bindungen erfolgt.

Eine Verringerung der Menge an Verunreinigungen durch Zersetzung durch das Reinigungsmaterial umfasst den Fall, dass bei der Zersetzung der Verunreinigungen Produkte entstehen, die ihrerseits nur noch in geringerem Maße, vorzugweise in geringem Maße, insbesondere überhaupt nicht mehr als Katalysatorgift für die verwendeten Katalysatoren unter den vorherrschenden Bedingungen fungieren. Eine Verringerung der Menge an Verunreinigungen durch deren Zersetzung durch das Reinigungsmaterial umfasst ebenfalls den Fall, dass bei der Zersetzung der Verunreinigungen Produkte entstehen, die an das Reinigungsmaterial über Adsorption und/oder Absorption gebunden werden können.

Das Reinigungsmaterial weist vorzugsweise eine Aufnahmekapazität für Verunreinigungen von bis zu 1 Gewichtsprozent, stärker bevorzugt bis zu 5 Gewichtsprozent, besonders bevorzugt bis zu 25 Gewichtsprozent, ganz besonders bevorzugt bis zu 40 Gewichtsprozent der Verunreinigungen, bezogen auf das Gewicht an verwendetem Reinigungsmaterial auf. Das Reinigungsmaterial weist vorzugsweise eine Aufnahmekapazität für schwefelhaltige Verunreinigungen, bezogen auf das Gewicht an verwendetem Reinigungsmaterial, von 500 ppmw oder mehr auf. Das Reinigungsmaterial weist vorzugsweise eine Aufnahmekapazität für schwermetallhaltige Verunreinigungen, bezogen auf das Gewicht an verwendetem Reinigungsmaterial, von 500 ppmw oder mehr auf. Die Aufnahmekapazität kann z.B. über die Massenbilanz des Eintrittsstroms und des Austrittsstroms in einem Durchbruchtest bestimmt werden. Dabei wird beispielsweise ein Eintrittsstrom mit einer bekannten Menge an Schwefelverunreinigung solange über das Reinigungsmaterial geleitet, bis im Austrittsstrom eine signifikante Menge der Schwefelverunreinigungen detektiert wird. Dies wird auch als Durchbruch bezeichnet. Aus der Volumenmenge, die pro Zeiteinheit über das Reinigungsmaterial geleitet wird, kann die Menge an Schwefel über einen gegebenen Zeitraum berechnet werden. Dieses Verfahren kann entsprechend auf andere Verunreinigungen, wie Schwermetallverunreinigungen oder vergleichbare Verunreinigungen angewendet werden.

Geeignete Reinigungsmaterialien können vorzugsweise ausgewählt werden aus der Gruppe bestehend aus Zink-basierten Reinigungsmaterialien, wie ZnO, Aluminiumbasierten Reinigungsmaterialien, wie Aluminiumoxid, vorzugsweise Al₂O₃, Siliziumbasierten Reinigungsmaterialien, wie Siliziumdioxid, AlSiOx-basierten Reinigungsmaterialien, wie Zeolithe, Aktivkohle, Tone, und Kupfer-basierten Reinigungsmaterialien. Zur Verringerung der Menge an Katalysatorgiften, insbesondere von Schwefel- und Chlorverbindungen eignen sich beispielsweise auch auf Aluminiumoxid geträgerte Oxide von Alkalimetallen, Erdalkalimetallen, Mangan, Yttrium oder Lanthan. Reinigungsmaterialien auf Basis von Aktivkohle oder Zeolithen sind beispielsweise in der EP 2 069 231 B1 zur Abtrennung von Metallcarbonylen aus einem Synthesegasstrom beschrieben.

Als Reinigungsmaterialen können prinzipiell auch die oben beschriebenen Methanolsynthese-Katalysatoren nach einer vollständigen oder teilweisen Deaktivierung verwendet werden. Dazu zählen beispielsweise Methanolsynthese-Katalysatoren, die bereits in einem Methanolsynthese-Reaktor verwendet wurden und hierdurch zumindest einen Teil ihrer Aktivität verloren haben. Vorzugsweise umfassen die Reinigungsmaterialen maximal 50 Gew.-%, bevorzugt maximal 10,0 Gew.-%, stärker bevorzugt maximal 2,0 Gew.-%, insbesondere 0,1 Gew.-% oder weniger, an Methanolsynthese-Katalysatoren nach einer vollständigen oder teilweisen Deaktivierung, bezogen auf das Gesamtgewicht der in den Reinigungsschichten vorhandenen Reinigungsmaterialen.

In einer bevorzugten Ausführungsform werden als Reinigungsmaterialen keine Methanolsynthese-Katalysatoren nach einer teilweisen Deaktivierung verwendet. In dieser Ausführungsform ist das Reinigungsmaterial im Wesentlichen inert in Bezug auf die Methanolsynthese, das heißt es trägt nicht wesentlich zur katalytischen Herstellung von Methanol bei. Vorzugsweise wird durch das Reinigungsmaterial 5 Volumenprozent oder weniger, stärker bevorzugt 1 Volumenprozent oder weniger, insbesondere 0,1 Volumenprozent oder weniger an Methanol gebildet, bezogen auf die Gesamtmenge an pro Zeiteinheit gebildetem Methanol, gemessen am Gasauslass.

Vorzugsweise wird durch das Reinigungsmaterial 5 Volumenprozent oder weniger an Nebenprodukten, bevorzugt 1 Volumenprozent oder weniger an Nebenprodukten, besonders bevorzugt 0,1 Volumenprozent oder weniger an Nebenprodukten gebildet, bezogen auf die Gesamtmenge an Gas am Gasauslass. Mögliche Nebenprodukte umfassen dabei Dimethylether, höher Alkohole oder höhere Kohlenwasserstoffe.

Die Reinigungsschichten können aus Reinigungsmaterial in Pulverform und/oder aus Reinigungsmaterial in Form von Formkörpern, insbesondere gepressten Formkörpern, wie zum Beispiel gepressten Tabletten oder gepressten Ringen, Pellets oder aus Extrudaten gebildet werden. Vorzugsweise sind die Reinigungsschichten aus Reinigungsmaterial in Pelletform ausgebildet. Bevorzugt ist das Reinigungsmaterial in den Reinigungsschicht(en) in Form von Festbett-Schüttungen angeordnet.

Gegenstand der Erfindung ist auch ein Verfahren zur katalytischen Herstellung von Methanol aus Synthesegas. Das Verfahren weist folgende Schritte auf:
(a) Bereitstellen eines Reaktors, vorzugsweise eines Wärmetauscher-Reaktors;
(b) Anordnen von zwei oder mehr als zwei Reaktionszonen im Reaktor,
(c) Beaufschlagen des Reaktors mit Synthesegas, umfassend Wasserstoff und Kohlenstoffoxide,
(d) Umsetzen des Synthesegases in dem Reaktor unter Methanolsynthesebedingungen zu Methanol, und
(e) Ausleiten des erzeugten Methanols und des nicht umgesetzten Synthesegases aus dem Reaktor,

wobei in den zwei oder den mehr als zwei Reaktionszonen jeweils wenigstens eine Katalysatorschicht angeordnet ist, und
wobei in den zwei oder den mehr als zwei Reaktionszonen jeweils wenigstens eine Reinigungsschicht angeordnet ist, die ein Reinigungsmaterial umfasst, und wobei die wenigstens eine Reinigungsschicht stromaufwärts der wenigstens einen Katalysatorschicht angeordnet ist.

Das Synthesegas umfasst üblicherweise eine Mischung aus Kohlenstoffmonoxid, Kohlenstoffdioxid und Wasserstoff.

Alternativ dazu kann im vorliegenden Verfahren zur katalytischen Herstellung von Methanol als Synthesegas auch eine Mischung aus Kohlenstoffdioxid und Wasserstoff verwendet werden. In diesem Fall werden die Kohlenstoffoxide im Synthesegas im Wesentlichen, d.h. zu 99 Vol.-% oder mehr, vorzugsweise zu 99.9 Vol.-% oder mehr, bezogen auf das Gesamtvolumen aller Kohlenstoffoxide im Synthesegasstrom, insbesondere ausschließlich aus Kohlenstoffdioxid gebildet. Aufgrund der Reversibilität der "Wassergas Shift"-Reaktion kann es dabei zu einer Bildung von Kohlenstoffmonoxid im Reaktor kommen, so dass eine Mischung aus Kohlenstoffmonoxid, Kohlenstoffdioxid und Wasserstoff *in situ* im Reaktor gebildet wird. Insbesondere bei einer Rückführung nicht umgesetzter Gasbestandteile (sogenannte Syntheseschleife) kann es dadurch zur Anreicherung von Kohlenstoffmonoxid kommen, das auf diese Weise in den Reaktor zurückgeführt werden kann.

In einer bevorzugten Ausführungsform sind die Reaktionszonen als Reaktorrohre ausgestaltet.

Vorzugsweise sind in den zwei oder den mehr als zwei Reaktionszonen, bei denen es sich vorzugsweise um Reaktorrohre handelt, jeweils wenigstens zwei voneinander verschiedene Katalysatorschichten angeordnet, wobei die erste Katalysatorschicht stromaufwärts angeordnet ist und die weitere(n) Katalysatorschicht(en) stromabwärts angeordnet ist/sind. In einer besonders bevorzugten Ausführungsform ist die Aktivität der ersten Katalysatorschicht niedriger als die Aktivität der weiteren Katalysatorschicht(en).

Vorzugsweise wird als Wärmetauschfluid Wasser bzw. Wasserdampf eingesetzt. Der im Verfahren bevorzugt verwendete Reaktor ist ein wassergekühlter Reaktor. Die Kühltemperatur liegt bevorzugt im Bereich von 180 °C bis 270 °C, vorzugsweise im Bereich von 200 °C bis 270 °C, stärker bevorzugt im Bereich von 210 °C bis 260 °C, insbesondere im Bereich von 220 °C bis 250 °C.

Beim Durchströmen des Synthesegases durch die Reinigungsschicht in den Reaktionszonen, bei denen es sich vorzugsweise um Reaktorrohre handelt, wird dieses vor Eintritt in die (erste) Katalysatorschicht vorgewärmt. Der Grad der Vorwärmung ist abhängig von der Eintrittstemperatur des Synthesegases, der Temperatur des Wärmetauschfluids und der Schichtdicke der Reinigungsschicht(en).

Die Eintrittstemperatur des Synthesegases am Gaseinlass des Reaktors liegt üblicherweise im Bereich von 150 °C bis 300 °C, vorzugsweise im Bereich von 170 °C bis 270 °C.

In einer bevorzugten Ausführungsform wird die Temperatur des Synthesegases durch die Verwendung einer Reinigungsschicht in den Reaktionszonen um einen Wert im Bereich von 0,1 K bis 50 K, vorzugsweise im Bereich von 5 K bis 40 K, insbesondere im Bereich von 10 K bis 30 K erhöht.

In einer bevorzugten Ausführungsform liegt die Temperatur des durch die Verwendung einer Reinigungsschicht in den Reaktionszonen vorgewärmten Synthesegases im Bereich von 180 °C bis 260 °C, vorzugsweise im Bereich von 200 °C bis 260 °C, stärker bevorzugt im Bereich von 210 °C bis 250 °C, insbesondere im Bereich von 220 °C bis 240 °C.

Die Erfindung wird im Folgenden unter Bezugnahme auf die beiliegenden Figuren durch mehrere Beispiele näher beschrieben. Die Figuren zeigen in:
- Fig. 1: illustrative nicht erfindungsgemäße erste Anordnung zweier Reaktorrohre ohne Reinigungsschicht mit einer Katalysatorschicht MM1
- Fig. 2: illustrative erfindungsgemäße zweite Anordnung zweier Reaktorrohre mit einer Reinigungsschicht und einer Katalysatorschicht MM1
- Fig. 3: illustrative erfindungsgemäße dritte Anordnung zweier Reaktorrohre mit einer Reinigungsschicht und zwei Katalysatorschichten MM1 und MM2
- Fig. 4: exemplarischen Temperaturverlauf für eine nicht erfindungsgemäße Anordnung ohne Reinigungsschicht mit einer Katalysatorschicht MM1
- Fig. 5: exemplarischen Temperaturverlauf für eine erfindungsgemäße Anordnung mit einer Reinigungsschicht und einer Katalysatorschicht MM1, wobei die vertikale gestrichelte Linie die Schichtgrenze zwischen Reinigungsschicht und Katalysatorschicht MM1 symbolisiert und die horizontale gestrichelte Linie die Temperatur des Wärmetauschfluids zeigt
- Fig. 6: exemplarischen Temperaturverlauf für eine erfindungsgemäße Anordnung mit einer Reinigungsschicht und zwei Katalysatorschichten MM1 und MM2, wobei beiden vertikalen gestrichelte Linien die Schichtgrenzen zwischen Reinigungsschicht und Katalysatorschicht MM1 bzw. zwischen Katalysatorschicht MM1 und Katalysatorschicht MM2 symbolisiert und die horizontale gestrichelte Linie die Temperatur des Wärmetauschfluids zeigt
- Fig. 7: exemplarische Temperaturverläufe für eine nicht erfindungsgemäße Anordnung ohne Reinigungsschicht mit einer Katalysatorschicht MM1 für verschiedene Nutzungsdauern
- Fig. 8: exemplarische Temperaturverläufe für eine erfindungsgemäße Anordnung mit einer Reinigungsschicht und einer Katalysatorschicht MM1 für verschiedene Nutzungsdauern
- Fig. 9: exemplarische Temperaturverläufe für eine erfindungsgemäße Anordnung mit einer Reinigungsschicht und zwei Katalysatorschichten MM1 und MM2 für verschiedene Nutzungsdauern
- Fig. 10: exemplarische Entwicklung der Methanolausbeute in Abhängigkeit von der Nutzungsdauer für eine erfindungsgemäße Anordnung mit einer Reinigungsschicht und einer Katalysatorschicht MM1 im Vergleich zu einer nicht erfindungsgemäßen Anordnung mit einer Katalysatorschicht MM1 aber ohne Reinigungsschicht unter Annahme einer durch die Reinigungsschicht um 20 % verlangsamten Deaktivierung (normiert auf die Ausgangs-Methanolausbeute der nicht erfindungsgemäßen Anordnung mit einer Katalysatorschicht MM1 aber ohne Reinigungsschicht)
- Fig. 11: exemplarische Entwicklung der Methanolausbeute in Abhängigkeit von der Nutzungsdauer für eine erfindungsgemäße Anordnung mit einer Reinigungsschicht und zwei Katalysatorschichten MM1 und MM2 im Vergleich zu einer nicht erfindungsgemäßen Anordnung mit einer Katalysatorschicht MM1 aber ohne Reinigungsschicht unter Annahme einer durch die Reinigungsschicht um 10 % und um 20 % verlangsamten Deaktivierung (normiert auf die Ausgangs-Methanolausbeute der nicht erfindungsgemäßen Anordnung mit einer Katalysatorschicht MM1 aber ohne Reinigungsschicht)

### Beispiele

In den nachfolgenden Beispielen wird ein kupferbasierter Katalysator der MegaMax^{®}-Serie mit unterschiedlichen Pelletgrößen verwendet und in den Beispielen und den Figuren als MM1 bzw. MM2 bezeichnet. MM1 weist eine Pelletgröße von 6 mm x 4 mm in zylindrischer Form (Durchmesser x Höhe) und MM2 eine Pelletgröße von 3 mm x 3 mm in zylindrischer Form (Durchmesser x Höhe) auf.

Die den Simulationen zugrundeliegende Versuchsanordnung wurde so gewählt, dass sie den Aufbau einer industriell typischen Anordnung einer Methanolsyntheseanlage wiedergibt. Der Reaktor ist als wassergekühlter Reaktor ausgebildet (WCR - Water Cooled Reactor) und wird mit Synthesegas bei einer Raumgeschwindigkeit von ca. 14,000 h⁻¹ durchströmt.

In den folgenden Beispielen und Figuren bezeichnet ferner TOS (time-on-stream) die Nutzungsdauer eines Katalysators in Jahren, sowie z die relative Position entlang der Reaktorachse in den Reaktorrohren in Strömungsrichtung unter Annahme einer 100 %-igen Füllung, wobei sich bei z gleich 0,0 der Gaseinlass und bei z gleich 1,0 der Gasauslass befindet. In den Figuren stellt die Strichlinie die Kühltemperatur dar. Die senkrechten Punktlinien verdeutlichen die verschiedenen Lagen.

### Beispiel 1 (Vergleich)

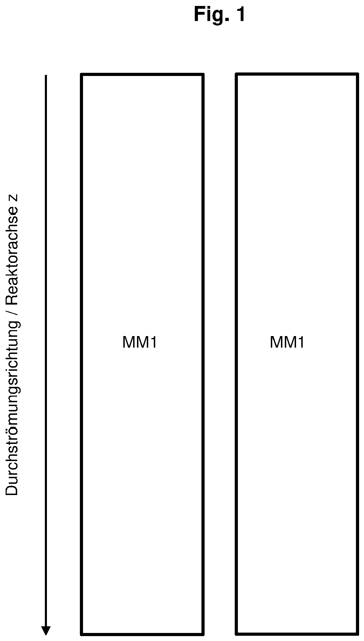

In Beispiel 1 wird ein wassergekühlter Methanolreaktor mit einer Beladung mit 100 Vol.-% einer Schüttung von Katalysator MM1 ohne stromaufwärts gelegene Reinigungsschicht verwendet. Dies entspricht dem in Fig. 1 abgebildeten Aufbau.

### Beispiel 2

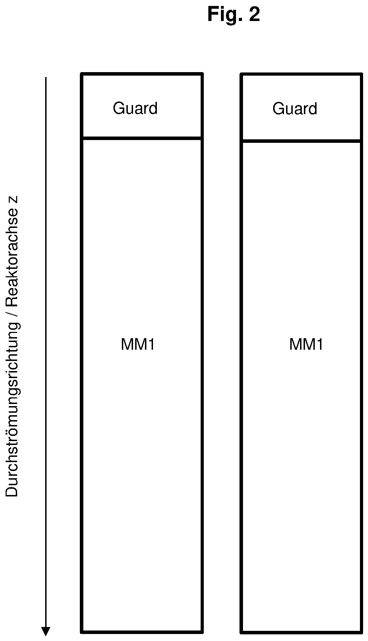

In Beispiel 2 wird ein wassergekühlter Methanolreaktor mit einer Beladung mit zwei Schichten (10 Vol.-% Guardbett, 90 Vol.-% Schüttung von Katalysator MM1) verwendet. Dies entspricht dem in Fig. 2 abgebildeten Aufbau.

### Beispiel 3

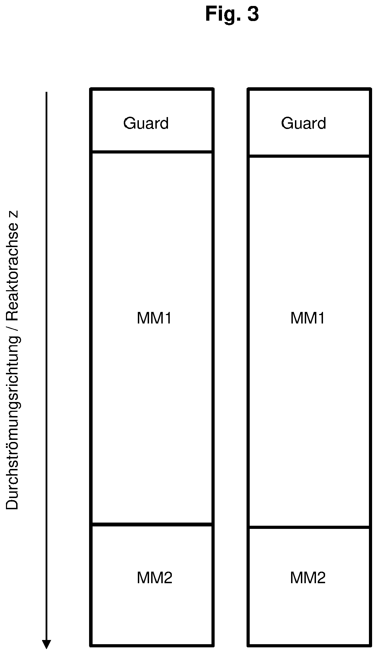

In Beispiel 3 wird ein wassergekühlter Methanolreaktor mit einer Beladung mit drei Schichten (10 Vol.-% Guardbett, 65 Vol.-% Schüttung von Katalysator MM1 und 25 Vol.-% Schüttung von Katalysator MM2) verwendet. Dies entspricht dem in Fig. 3 abgebildeten Aufbau.

### Beispiel 4

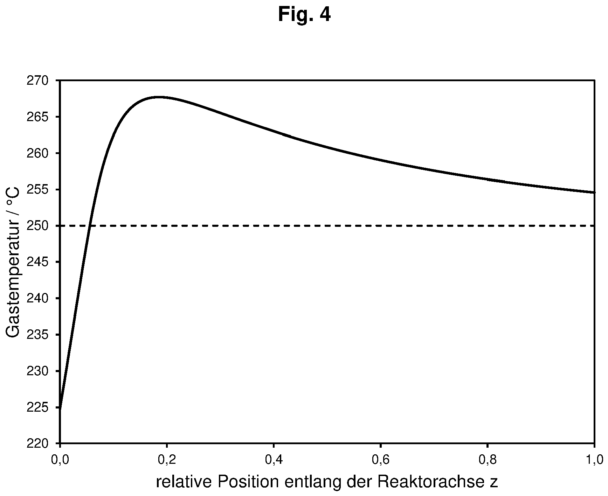

Fig. 4 zeigt einen typischen Temperaturverlauf im wassergekühlten Methanolreaktor gemäß Beispiel 1 mit einer Kühltemperatur von 250 °C in Abhängigkeit von der Position entlang der Reaktorachse z.

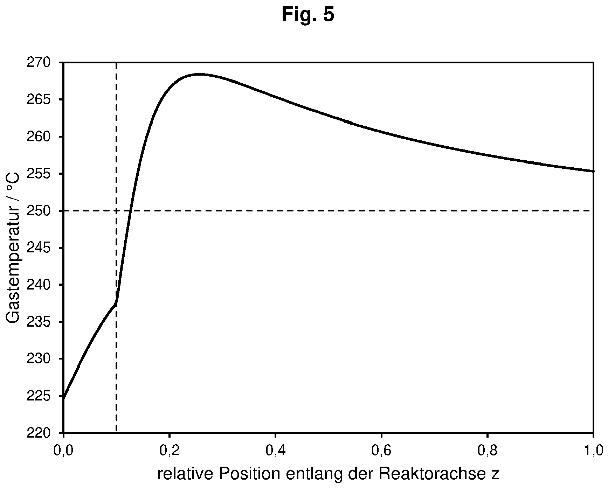

Fig. 5 zeigt einen typischen Temperaturverlauf im wassergekühlten Methanolreaktor gemäß Beispiel 2 mit einer Kühltemperatur von 250°C in Abhängigkeit von der Position entlang der Reaktorachse z. Das Guardbett wurde als in Bezug auf die Methanolproduktion inerte Schüttung aus Kugeln mit einem Durchmesser von 3 mm angenommen.

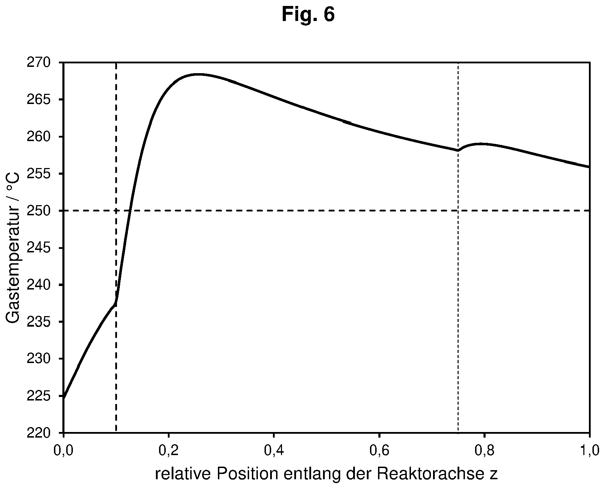

Fig. 6 zeigt einen typischen Temperaturverlauf im wassergekühlten Methanolreaktor gemäß Beispiel 3 in Abhängigkeit von der Position entlang der Reaktorachse z.

Der Vergleich von Fig. 4 mit Fig. 5 zeigt, dass die Anwesenheit einer stromaufwärts angeordneten Reinigungsschicht zu einer signifikanten Erwärmung des eintretenden Gases von ca. 225 °C auf ca. 238 °C, d.h. um etwa 13 K, führt. Trotz dieser Erwärmung erhöht sich die Hot-Spot-Temperatur nur um weniger als 1 K von ca. 267 °C auf ca. 268 °C. Durch die Aufheizung des Gases in der (für die Methanolreaktion inerten) Guardbettschicht erreicht das Synthesegas den Katalysator bei einer für seine Aktivität vorteilhaften höheren Temperatur.

In Fig. 6 ist zusätzlich ein zweiter Hot-Spot in der zweiten (aktiveren) Katalysatorschicht zu sehen. Der Hot-Spot ist das Resultat der durch die zusätzliche Reaktion entstehenden Exothermie, die durch die höhere Aktivität der zweiten Katalysatorschicht zustande kommt, und illustriert somit eine weitere Steigerung der Ausbeute im Vergleich zu Beispiel 2 in Fig. 5. Der Vergleich von Fig. 6 mit Fig. 5 zeigt, dass die Anwesenheit einer zweiten aktiveren Katalysatorschicht zu einer Erwärmung des Gases um etwa 2 K führt, die aus einer höheren Umsatzrate durch diesen Katalysator resultiert.

### Beispiel 5

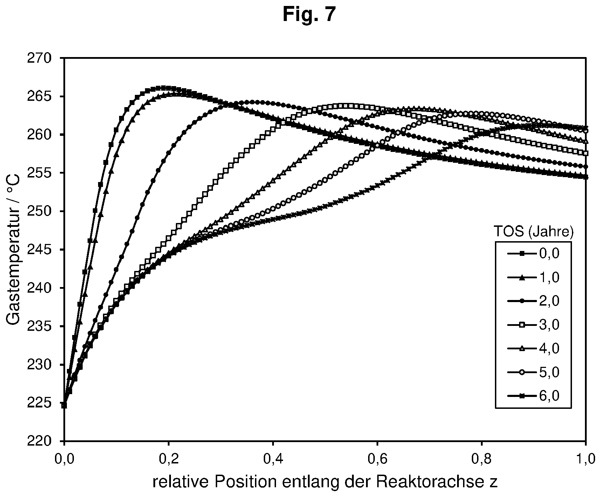

Fig. 7 zeigt typische Temperaturverläufe in dem wassergekühlten Methanolreaktor gemäß Beispiel 1 in Abhängigkeit von der Position entlang der Reaktorachse z für Nutzungsdauern (TOS = time-on-stream) von 0 bis 6 Jahren. Das zugrundeliegende Deaktivierungsprofil wurde aus realen Anlagendaten abgeleitet. Die Deaktivierung schreitet mit der Zeit entlang der Reaktorachse z fort und wird mathematisch durch eine sogenannte logistische Funktion beschrieben. Dies führt zu der in realen Anlagen beobachteten Verschiebung des Hot-Spots stromabwärts über die Nutzungsdauer.

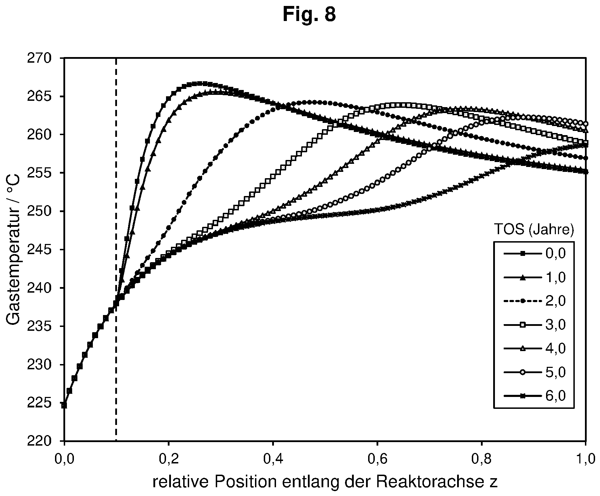

Fig. 8 zeigt typische Temperaturverläufe in dem wassergekühlten Methanolreaktor gemäß Beispiel 2 in Abhängigkeit von der Position entlang der Reaktorachse z für Nutzungsdauern (TOS = time-on-stream) von 0 bis 6 Jahren. Das Guardbett wurde jeweils als in Bezug auf die Methanolproduktion inerte Schüttung aus Kugeln mit einem Durchmesser von 3 mm angenommen. In der Simulation wurde dasselbe Deaktivierungsverhalten zugrunde gelegt wie in Fig. 7 (d.h. eine zu erwartende verringerte Alterung aufgrund der Verringerung der Menge an Katalysatorgiften im Synthesegas aufgrund des Guardbetts wurde nicht berücksichtigt). Die Simulationen spiegeln daher ein "worst-case" Szenario wider, die lediglich den positiven Einfluss der erfindungsgemäßen Kombination aus Guardbettschicht und Katalysatorschichtung auf den Temperaturverlauf im Reaktorrohr über die Lebensdauer demonstrieren.

Der Vergleich von Fig. 7 und Fig. 8 zeigt, wie schon zuvor, dass zum einen die Anwesenheit einer stromaufwärts angeordneten Reinigungsschicht zu einer signifikanten Erwärmung des eintretenden Gases von ca. 225 °C auf ca. 238 °C, d.h. um etwa 13 K beiträgt. Trotz dieser Erwärmung erhöht sich die Hot-Spot-Temperatur nur um weniger als 1 K von ca. 267 °C auf ca. 268 °C. Durch die Aufheizung des Gases in der (für die Methanolreaktion inerten) Guardbettschicht erreicht das Synthesegas den Katalysator bei einer für seine Aktivität vorteilhaften höheren Temperatur.

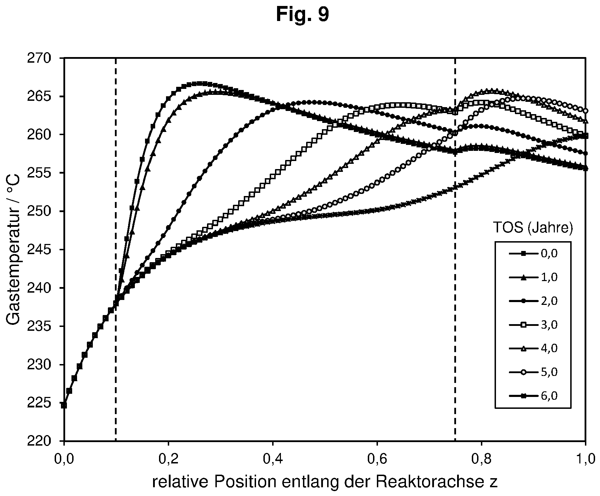

In Fig. 9 ist wieder ein zweiter Hot-Spot in der zweiten (aktiveren) Katalysatorschicht zu sehen. Der Vergleich von Fig. 9 mit Fig. 8 zeigt, dass die Anwesenheit der zweiten Katalysatorschicht mit einem aktiveren Katalysator beispielsweise bei einer Nutzungsdauer von 2 Jahren immer noch zu einer signifikanten Erwärmung des Gases um etwa 3 K aufgrund einer höheren Umsatzrate durch den aktiveren Katalysator führt.

### Beispiel 6

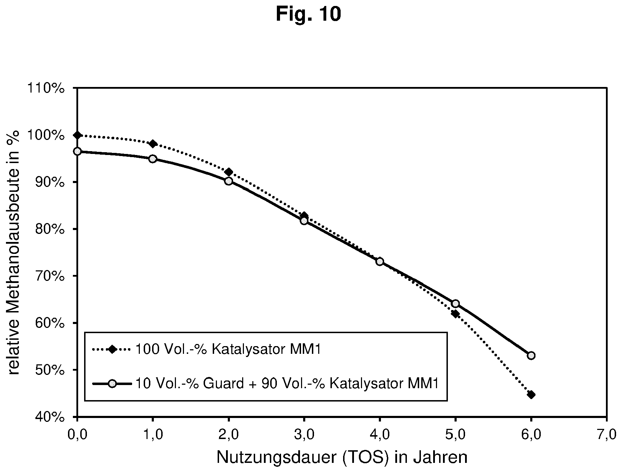

Fig. 10 zeigt die relative Methanolausbeute in Prozent in Abhängigkeit von der Nutzungsdauer für eine erfindungsgemäße Anordnung mit zwei Schichten gemäß Beispiel 2 (10 Vol.-% Guardbett und 90 Vol.-% einer Schüttung von Katalysators MM1) im Vergleich zu einer nicht erfindungsgemäßen Anordnung ohne stromaufwärts gelegene Reinigungsschicht gemäß Beispiel 1 (100 Vol.-% Schüttung von Katalysator MM1). Die Werte sind normiert auf die Methanolausbeute einer 100 Vol.-% Schüttung von Katalysator MM1 zum Zeitpunkt 0 Jahre. In den Simulationen wurde für die erfindungsgemäße Anordnung eine um 20 % verringerte Deaktivierung aufgrund der Verringerung der Menge an Katalysatorgiften im Synthesegas durch das

Reinigungsmaterial im Guardbett angenommen. Fig. 10 zeigt eine langsamere Abnahme der Methanolausbeute für die erfindungsgemäße Anordnung. Dieser Unterschied des erfindungsgemäßen Reaktors im Vergleich zu einem konventionellen Reaktor zeigt sich insbesondere ab einer Laufzeit von 4 Jahren, ab der sich eine deutlich höhere Methanolausbeute manifestiert.

### Beispiel 7

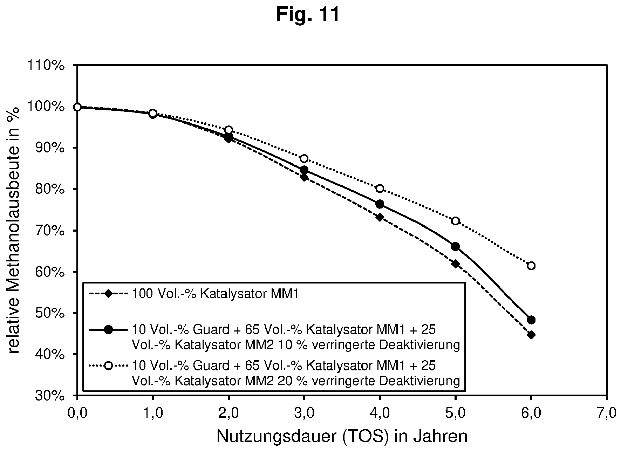

Fig. 11 zeigt die relative Methanolausbeute in Prozent in Abhängigkeit von der Nutzungsdauer für eine erfindungsgemäße Anordnung mit drei Schichten gemäß Beispiel 3 (10 Vol.-% Guardbett, 65 Vol.-% Schüttung von Katalysator MM1 und 25 Vol.-% Schüttung von Katalysator MM2) im Vergleich zu einer nicht erfindungsgemäßen Anordnung ohne stromaufwärts gelegene Reinigungsschicht gemäß Beispiel 1 (100 Vol.-% Schüttung von Katalysator MM1). Die Werte sind normiert auf die Methanolausbeute einer 100 Vol.-% Schüttung von Katalysator MM1 zum Zeitpunkt 0 Jahre. In den Simulationen wurde für die erfindungsgemäße Anordnung eine um 10% und eine um 20 % verringerte Deaktivierung aufgrund der Verringerung der Menge an Katalysatorgiften im Synthesegas durch das Reinigungsmaterial im Guardbett angenommen. Fig. 11 zeigt, dass durch die Anordnung unterschiedlich aktiver Katalysatorschichten eine identische Anfangsausbeute an Methanol erzielt wird wie im Vergleichsbeispiel, obwohl nur 90 % des Reaktors mit Katalysator befüllt sind (dies entspricht also einer höheren Raum-Zeit-Ausbeute) bei gleichzeitiger Einbringung einer Reinigungsschicht. Die langsamere Abnahme der Methanolausbeute für die erfindungsgemäße Anordnung führt von Beginn an und über die gesamte Lebensdauer zu einer höheren Methanolausbeute im Vergleich zu einer nicht erfindungsgemäßen Anordnung.

Die oben beschriebenen exemplarischen Ausführungsformen sind nicht als limitierend zu verstehen. Andere Ausführungsformen, die konsistent mit den oben beschriebenen exemplarischen Ausführungsformen sind, sind nunmehr für den Fachmann offenkundig beschrieben.

## Patentansprüche

1. Reaktor zur katalytischen Herstellung von Methanol, umfassend zwei oder mehr als zwei Reaktionszonen, die von einem Wärmetauschfluid umgeben sind, wobei
in den zwei oder den mehr als zwei Reaktionszonen jeweils wenigstens eine Katalysatorschicht angeordnet ist und
in den zwei oder den mehr als zwei Reaktionszonen jeweils wenigstens eine Reinigungsschicht angeordnet ist, die ein Reinigungsmaterial umfasst, und wobei die wenigstens eine Reinigungsschicht stromaufwärts der wenigstens einen Katalysatorschicht angeordnet ist.

2. Reaktor nach Anspruch 1, wobei in den zwei oder den mehr als zwei Reaktionszonen jeweils wenigstens zwei voneinander verschiedene Katalysatorschichten angeordnet sind, wobei die erste Katalysatorschicht stromaufwärts angeordnet ist und die weitere(n) Katalysatorschicht(en) stromabwärts angeordnet ist/sind.

3. Reaktor nach Anspruch 2, wobei die Aktivität der ersten Katalysatorschicht niedriger ist als die Aktivität der weiteren Katalysatorschicht(en).

4. Reaktor nach einem der Ansprüche 1, 2 oder 3, wobei die Schichthöhe der Reinigungsschicht(en) 5 bis 30 %, stärker bevorzugt 7,5 bis 20 %, besonders bevorzugt 10 bis 15 % der Gesamtschichthöhe aller Katalysatorschicht(en) und Reinigungsschicht(en) in den Reaktionszonen beträgt.

5. Reaktor nach einem der Ansprüche 1 bis 4, wobei eine zusätzliche Reinigungsschicht stromaufwärts der zwei oder mehr als zwei Reaktionszonen, vorzugsweise innerhalb des Reaktors, angeordnet ist, wobei die zusätzliche Reinigungsschicht Reinigungsmaterial aufweist, das gleich oder verschieden ist von dem Reinigungsmaterial in den zwei oder den mehr als zwei Reaktionszonen.

6. Reaktor nach einem der Ansprüche 1 bis 5, wobei das Reinigungsmaterial im Wesentlichen nicht zur katalytischen Herstellung von Methanol beiträgt.

7. Reaktor nach einem der Ansprüche 1 bis 6, wobei das Reinigungsmaterial in der Lage ist, die Menge an Verunreinigungen, die in dem in den Reaktor eingebrachten Synthesegasstrom vorhanden sind und den Katalysator vergiften können, insbesondere Metalle und deren Verbindungen, schwefelhaltige Verbindungen, ungesättigte Kohlenwasserstoffe, und/oder chlorhaltige Verbindungen, insbesondere elementares Eisen, Eisen-Carbonyle, Nickel-Carbonyle oder Schwefelwasserstoffe, über Adsorption und/oder Absorption und/oder Zersetzung zu verringern.

8. Reaktor nach einem der Ansprüche 1 bis 7, wobei die Katalysatoren in den Katalysatorschicht(en) und/oder das Reinigungsmaterial in den Reinigungsschicht(en) in Form von Festbett-Schüttungen angeordnet sind.

9. Reaktor nach einem der Ansprüche 1 bis 8, wobei die zwei oder mehr als zwei Reaktionszonen in einem Wärmetauscher-Reaktor angeordnet sind, wobei die Reaktionszonen im Wesentlichen parallel und/oder radial zur zentralen Längsachse des Reaktors angeordnet sind und der Reaktor als wassergekühlter, ölgekühlter oder als gasgekühlter Reaktor, vorzugsweise als wassergekühlter Reaktor ausgebildet ist.

10. Reaktor nach einem der Ansprüche 1 bis 9, wobei die Reaktionszonen als Reaktorrohre ausgebildet sind.

11. Verfahren zur katalytischen Herstellung von Methanol aus Synthesegas, wobei das Verfahren folgende Schritte umfasst:
(a) Bereitstellen eines Reaktors, vorzugsweise eines Wärmetauscher-Reaktors;
(b) Anordnen von zwei oder mehr als zwei Reaktionszonen, ausgewählt aus Reaktorrohren oder Reaktorplatten, die von einem Wärmetauschfluid umgeben sind, im Reaktor,
(c) Beaufschlagen des Reaktors mit Synthesegas, umfassend Wasserstoff und Kohlenstoffoxide,
(d) Umsetzen des Synthesegases in dem Reaktor unter Methanolsynthesebedingungen zu Methanol, und
(e) Ausleiten des erzeugten Methanols und des nicht umgesetzten Synthesegases aus dem Reaktor,
wobei in den zwei oder den mehr als zwei Reaktionszonen jeweils wenigstens eine Katalysatorschicht angeordnet ist, und
wobei in den zwei oder den mehr als zwei Reaktionszonen jeweils wenigstens eine Reinigungsschicht angeordnet ist, die ein Reinigungsmaterial umfasst, und wobei die wenigstens eine Reinigungsschicht stromaufwärts der wenigstens einen Katalysatorschicht angeordnet ist und
wobei die Reaktionszonen vorzugsweise als Reaktorrohre ausgebildet sind.

12. Verfahren nach Anspruch 11, wobei in den zwei oder den mehr als zwei Reaktionszonen jeweils wenigstens zwei voneinander verschiedene Katalysatorschichten angeordnet sind, wobei die erste Katalysatorschicht stromaufwärts angeordnet ist und die weitere(n) Katalysatorschicht(en) stromabwärts angeordnet ist/sind.

13. Verfahren nach Anspruch 12, wobei die Aktivität der ersten Katalysatorschicht niedriger ist als die Aktivität der weiteren Katalysatorschicht(en), wobei vorzugsweise die Aktivität jeder vorangehenden Katalysatorschicht jeweils niedriger ist als die Aktivität der jeweils nachfolgenden Katalysatorschicht(en).

14. Verfahren nach einem der Ansprüche 11, 12 und 13, wobei der Reaktor ein wassergekühlter Reaktor ist und die Kühltemperatur im Bereich von 180 °C bis 270 °C, vorzugsweise im Bereich von 210 °C bis 260 °C, insbesondere im Bereich von 220 °C bis 250 °C liegt.
